Europäisches Patentamt

European Patent Office

Office européen des brevets

(19)

(11) Publication number: **0 295 526 B1**

(12) **EUROPEAN PATENT SPECIFICATION**

(45) Date of publication of patent specification: **22.04.92**

(51) Int. Cl.⁵: **G01N 33/52**, C12Q 1/56, //G01N33/70,G01N33/72

(21) Application number: **88109015.3**

(22) Date of filing: **06.06.88**

(54) Process and device for separating and testing whole blood.

(30) Priority: **19.06.87 US 63680**

(43) Date of publication of application:
**21.12.88 Bulletin 88/51**

(45) Publication of the grant of the patent:
**22.04.92 Bulletin 92/17**

(84) Designated Contracting States:
**DE FR GB**

(56) References cited:
**EP-A- 0 159 727**
**EP-A- 0 182 373**
**DE-A- 3 441 149**
**US-A- 3 552 928**
**US-A- 4 111 657**

(73) Proprietor: **MILES INC.**
**1127 Myrtle Street**
**Elkhart Indiana 46514(US)**

(72) Inventor: **Genshaw, Marvin A.**
**2905 Neff Street**
**Elkhart, IN(US)**
Inventor: **Stover, Lon R.**
**50903 County Road 7 N**
**Elkhart, IN(US)**

(74) Representative: **Dänner, Klaus, Dr. et al**
**c/o Bayer AG Konzernverwaltung RP Patentabteilung**
**W-5090 Leverkusen 1 Bayerwerk(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

FIELD OF THE INVENTION

The present invention relates to a process and device for separating plasma or serum from undiluted whole blood. More particularly, the present invention relates to an improved method of removing the cellular components from whole blood by utilizing a bibulous matrix impregnated with a separating reagent. The essentially cell-free plasma or serum, in an undiluted state, then is directed to a test reagent-impregnated assay area of the same or adjacent bibulous matrix for immediate detection of one or more constituents of the separated soluble plasma or serum.

BACKGROUND OF THE INVENTION

Presently, there are numerous test devices available to simply and rapidly analyze body fluids for the presence or absence of a particular soluble constituent. For example, tests are available to detect glucose, uric acid or protein in urine, or to detect glucose, triglycerides, potassium ion or cholesterol in blood. Historically, the most difficult tests to design are those intended to analyze whole blood for a particular soluble constituent.

The red blood cells are the primary interfering substance in whole blood. Most simple blood tests are chromogenic, whereby a soluble constituent of the whole reacts with a particular reagent either to form a uniquely-colored complex or derivative as a qualitative indication of the constituent's presence or absence, or to form a colored complex or derivative of variable color intensity as a quantitive indication of the presence of the constituent. The deep red coloration of the whole blood seriously interferes with these chromogenic tests, and necessitates the removal of these highly-colored cells before the blood is tested.

Other cellular material, including both red and white blood cells, also can interfere in these chromogenic tests. However, most of this cellular material is removed during the separation of the red blood cells from the plasma or serum and, therefore, the interfering cellular material is not present in sufficient quantity to provide substantial background interference. The presence of red blood cells also can interfere with some nonchromogenic blood tests, whereby the tests results are either inconsistent or, if consistent, give assay results which are not equal to the true value. Therefore, it is essential to separate the serum or plasma from whole blood prior to analyzing the whole blood for soluble components.

Conventionally, the plasma or serum is separated from the cellular material by centrifugation. The cellular material collects at the bottom of the centrifuge tube and the supernatant plasma or serum is decanted. This process has the major disadvantages of requiring a relatively large blood sample, usually 0.1 ml to 5.0 ml, and a long centrifuge time of approximately 20 minutes.

The centrifuge method is suitable for large, automated laboratories that assay a multitude of blood samples, and for institutions, such as hospitals, not requiring the assay results in a matter of minutes. However, many small or private medical offices do not have such a blood separator on site. Therefore, simple chromogenic tests cannot be performed on site and the whole blood must be sent elsewhere for separation and assay. As a result, the assay results are not available in minutes but in hours or days.

Methods other than centrifugation have been used to separate the serum or plasma from the red blood cells of small whole blood samples. One of the simpler methods, as disclosed by Adams et al in U.S. Patent No. 3,092,465, uses a bibulous, or moisture absorbing, matrix that is impregnated with a testing reagent and coated with a semi-permeable barrier. The barrier screens out the cellular material and permits passage of the smaller molecules and ions to the testing reagent-impregnated bibulous matrix. In the case of a positive test, the relatively clear plasma or serum will react with the chromogenic testing reagent to color the bibulous matrix. The color is observed by simply rinsing or wiping away the screened out cells with water. Unfortunately, this technique is somewhat cumbersome and laborious.

Fetter in U.S. Patent Nos. 3,552,925 and 3,552,928 discloses another method and device to test small whole blood samples for soluble constituents. Fetter describes a bibulous matrix impregnated with a nonvolatile inorganic salt or an amino acid at one location and a test reagent at an adjacent location on the matrix. Blood is introduced onto the bibulous matrix such that the blood first contacts the inorganic salt or amino acid. The salt or amino acid precipitates the cellular material from the blood, and the plasma or serum then migrates to the reagent-impregnated portion of the matrix for chromogenic reaction. The salts or amino acids used in this process effectively separates the red blood cells from the whole blood, but also introduces contaminating ions or molecules into the plasma or serum, and also precipitates a portion of the soluble constituents, therefore making quantitative analysis of these constituents unreliable. The Fetter method obviates the need of a distinct separation step. However, the problem with this method is that the water portion of the blood no longer contains the constituents of interest.

U.S. Patent No. 4,477,575, Vogel et al, discloses a process and composition, including glass fibers having a defined average diameter and density, for separating plasma or serum from whole blood. In addition to the defined glass fiber parameters, the amount of plasma or serum to be separated is limited to at most 50%, and preferably less than 30%, of the absorption volume of the glass fibers. Otherwise, whole blood, containing approximately 50% filterable cellular material, will effectively clog the glass fiber layer. Therefore, a high ratio of hydrophobic glass fibers to whole blood volume is required.

Whole blood usually is diluted before assaying for a soluble plasma or serum constituent. This dilution not only requires an extra manipulative step, but also introduces a possibility of error due to incorrect dilution of the blood sample. In German Patent No. 3,441,149 there is disclosed a method of separating plasma or serum from whole blood by passing the blood through a lectin-impregnated matrix that is repeatedly rinsed with a diluent to dilute the plasma or serum before assay. The possibility of imprecise dilution is high, leading to an incorrect assay of the plasma or serum constituent.

EP-A2 159 727 describes a multi layer analytical element for analysis of whole blood sample. This element needs a blood cell filtering layer.

In developing a process and device for separating and testing small samples of whole blood, a number of considerations arise. One consideration is the degree of sophistication of the technician performing the assay. It is often desirable to have relatively untrained personnel carry out routine assays and obtain accurate quantitative results. In these situations, it is important that the assay method contain a minimum of manipulative steps, be free of possible interferences or contamination and provide for easy measurement. For instance, among the several possible manipulative steps, the dilution of the whole blood, or plasma or serum, prior to testing introduces the most probable step for assay error.

Therefore, a need exists for a process and device to efficiently and accurately separate and test small volumes of whole blood. The process should avoid a distinct manipulative step to separate the cellular material from the plasma or serum prior to assay, and, in order to avoid dilution errors, the process should allow an assay on the undiluted plasma Or serum. It is also desirable to find a blood separation and testing method that avoids the time delays of the present methods and yields accurate and reproducible results. The ideal process includes withdrawing a whole blood sample in "noninvasive" amounts, such as a pin prick drop, and immediately depositing the undiluted whole blood sample on a combination separating-analyzing device, whereby the undiluted plasma or serum is separated from the cellular components, and the presence and/or amount of a plasma or serum constituent is determined within minutes. Such a separation and testing method and device would allow medical personnel to carry out whole blood analyses on a more routine and more confident basis.

## SUMMARY OF THE INVENTION

In brief, the present invention is directed to a process and device for separating serum or plasma from undiluted whole blood, such that the undiluted serum or plasma is available for further testing, such as assay of soluble constituents, without any additional manipulative steps. It has been found that separating the plasma or serum from the cellular components of whole blood by the method and device of the present invention unexpectedly provides an undiluted serum or plasma sample containing essentially only the soluble plasma or serum constituents present in whole blood. The separation method and device neither introduces unacceptable contaminants into the serum or plasma nor alter the compositional makeup of the soluble plasma or serum.

In accordance with the present invention, the separation and testing device includes one or more hydrophilic, bibulous matrices, treated to include a separating reagent such that the serum or plasma is separated from the cellular blood components. The separated serum or plasma, in an undiluted form, then can be further treated or tested. For example, it can be assayed for a particular soluble constituent. The process includes depositing a whole blood sample onto a hydrophilic, bibulous matrix that has been impregnated with a separating reagent. As used here, and hereinafter, the expression "separating reagent" is defined as a chemical or mixture of chemicals that effects a separation of the red blood cells from the remaining soluble blood constituents. Likewise, as used here and hereinafter, the expression "test reagent" is defined as a chemical or mixture of chemicals causing an observable or detectable reaction when contacted with the substance being detected. The whole blood passes through the impregnated bibulous matrix, whereby the red blood cells are removed from the plasma or serum through the action of the separating reagent. In accordance with one important embodiment of the present invention, the undiluted plasma or serum continues to pass to an assay area of the same, or an adjacent, bibulous matrix, containing a suitable test reagent, to perform the assay of interest. The undiluted plasma or serum reacts with the test reagent in the assay region to produce a detectable change in the assay region, such as a color change, to chromogenically indicate the presence or absence of a particular soluble constituent and/or to allow quantitative determination of the particular soluble constituent.

Therefore, the present invention is directed to a method and device for rapidly and effectively separating the soluble constituents of plasma or serum from undiluted whole blood samples. More particularly, in accordance with another important feature of the present invention, one or more chemically-treated, hydrophilic bibulous matrices are so arranged so as to separate the serum or plasma from whole blood, and then immediately test the serum or plasma qualitatively or quantitatively for soluble constituents. The whole blood is deposited on an area of the bibulous matrix that has been treated with a thrombin or preferably certain selected lectins. This area of the bibulous matrix removes the cellular components of the whole blood without adding contaminating ions or molecules or removing soluble constituents from the serum or plasma. This lectin concentration is preferably between about 55 and about 40,000 units per cm$^3$ of matrix, wherein each unit is a micogram of lectin necessary to agglutinate a two percent solution of red blood cells within one hour of incubation at 25° C. The thrombin concentration is preferably between about 90 and about 900 NIH (National Institute of Health) units per cm$^3$ of matrix. The undiluted serum or plasma can pass through the separation area of the matrix to an assay area of the same bibulous matrix, or to a second, adjacent bibulous matrix for assay. In either case, the assay area has been previously treated to include a testing reagent such that the particular soluble plasma or serum constituent of interest can be detected immediately without dilution or other manipulative steps.

Therefore, it is an object of the present invention to provide a method and device to separate the soluble constituents from small quantities of undiluted whole blood quickly, effectively and accurately.

It is also an object of the present invention to provide a method and device for the rapid, convenient and effective separation of plasma or serum from the cellular components of undiluted whole blood.

Another object of the present invention is to provide a method and device to assay the plasma or serum separated from the undiluted whole blood for soluble constituents without any added manipulative steps. In other words, the object of the present invention is to provide a material and device to separate and test the plasma or serum without diluting the whole blood, plasma or serum.

Another object of the present invention is to provide a method and device to test undiluted plasma or serum for soluble constituents.

Another object of the present invention is to provide a method and device to test undiluted plasma or serum for soluble components, wherein the nature and the amount of the soluble constituents are not altered by the separation process.

Another object of the present invention is to provide a method for separating undiluted whole blood by utilizing an agglutinizing agent or a coagulating agent.

Another object of the present invention is to provide a method for separating undiluted whole blood by utilizing a lectin or a thrombin.

## BRIEF DESCRIPTION OF THE DRAWING

The above and other objects and advantages of the present invention will become apparent from the following detailed description of the present invention taken in conjunction with the drawing, wherein:

FIG. 1 is a perspective view of a device for separating plasma or serum from whole blood;

FIGS. 2 and 3 are perspective views of a device constructed in accordance with an embodiment of the present invention for separating plasma or serum from whole blood having a separating matrix as shown in FIG. 1, and then testing the plasma or serum qualitatively or quantitatively for a predetermined component of the plasma or serum in a second, testing matrix;

FIGS. 4 through 10 are views similar to FIGS. 2 and 3 showing blood separating and testing devices having a barrier between blood separating and testing portions to prevent blood spillover from a separating matrix onto a testing matrix;

FIGS. 6 and 7 are views similar to FIGS. 2-5 showing the separating and testing matrices physically separated and connected by a thin, absorbent tissue bridge;

FIGS. 8 and 9 are views similar to FIGS. 2-7 showing alternative configurations of the device of the present invention; and

FIG. 10 is a view similar to FIGS. 2-9 showing another construction of the device of the present invention including only one bibulous matrix.

## DETAILED DESCRIPTION OF THE INVENTION

In accordance with the present invention, plasma or serum is separated from undiluted whole blood samples, so that the undiluted plasma or serum is available for further treatment, such as immediate testing for particular soluble constituents. According to the method and device of the present invention, a small blood sample, usually a pin prick amount, is sufficient for separation and assaying, as opposed to the large milliliter size blood samples required in the centrifuge method of separation. Unexpectedly, the highly-colored red blood cells are separated from the plasma or serum, and the plasma or serum is assayed for a particular soluble constituent within minutes, without the additional manipulative steps of centrifugation, decantation and plasma or serum dilution. Surprisingly, the process and device of the present invention provide a rapid, economical way of performing whole blood assays on undiluted plasma or serum samples that are not contaminated by the separation process. Overall, the process and device of the present invention are ideally suited for routine blood assays in small laboratories or private physician offices, wherein the number of assays may be relatively low, but accurate results are still required in a short time period.

As will become apparent from the following detailed description of the invention, the method and device of the present invention are especially suited for blood assays utilizing chromogenic responses to test for the presence, absence or concentration of various soluble constituents of whole blood. Therefore, it is of primary importance to remove the highly-colored red blood cells from the whole blood sample in order for the chromogenic reaction to be detected and measured. Any method or device used to separate the red blood cells from the plasma or serum has several inherent constraints including fast and efficient cell removal; removal of only the cellular components and not the soluble plasma constituents; not contaminating the plasma or serum with interfering, soluble ions or molecules; and minimizing or eliminating hemolysis, wherein the red blood cells rupture and release their highly-colored components to the plasma or serum.

In accordance with the present invention, it has been found that red blood cells are effectively separated from the essentially colorless plasma or serum by the whole blood sample permeating through a suitable bibulous matrix that has been impregnated with, or otherwise contains, a suitable separating reagent. The separating reagent promotes the removal of the highly-colored cells from the fluid in which they are suspended, to yield a straw-colored plasma or serum that is available for simple determination of its soluble components.

Generally, the bibulous matrix of the present invention can be any hydrophilic, absorbent matrix that is amenable to treatment with a red blood cell separating reagent. The bibulous matrix also must permit the whole blood to permeate through at such a rate to allow time for red blood cell separation, yet rapidly enough to obtain blood assays quickly. In addition, the bibulous matrix must not promote hemolysis, contaminate the serum or plasma by serum or plasma-extraction of components of the bibulous matrix, remove serum or plasma constituents by chemical or physical interactions, or appreciably alter the undiluted plasma or serum in a way to make the subsequent blood assays inconclusive, inaccurate or doubtful.

The bibulous matrix of the present invention is a hydrophilic material, possessing the above-mentioned characteristics, that allows the blood to move, in response to capillary forces, through the matrix. The red blood cells are separated from the plasma or serum and retained by the bibulous matrix, and the essentially unchanged serum or plasma continues migrating through the bibulous matrix to an assay area of the separating and testing device.

The bibulous matrix can be any hydrophilic material that allows only the straw-colored plasma or serum to pass through to contact the assay area for analysis. Suitable bibulous matrices include hydrophilic inorganic powders, such as silica gel, alumina, diatomaceous earth and the like; sponge materials; argillaceous substances; cloth; hydrophilic natural polymeric materials, particularly cellulosic material, like cellulosic beads, and especially fiber-containing papers such as filter paper or chromatographic paper; synthetic or modified naturally-occuring polymers, such as cellulose acetate, polyvinyl chloride, polyacrylamide, polyacrylates, polyurethanes, crosslinked dextran, agarose, and other such crosslinked and noncrosslinked water-insoluble hydrophilic polymers. Hydrophobic substances, such as a hard, porous plastic, are not suitable for use as the bibulous matrix of the present invention.

The bibulous matrices can have different physical characteristics and can be of different chemical compositions or a mixture of chemical compositions. The matrix can also vary in regards to smoothness and roughness combined with hardness and softness. However, in every instance, the bibulous matrix must include a hydrophilic material. Regardless of the exact composition of the bibulous matrix, the primary considerations are separation and collection of the red blood cells and transmittal of substantially unchanged, undiluted plasma or serum.

To achieve the full advantage of the present invention, the hydrophilic, bibulous matrix includes a cellulosic material, such as paper, and preferably filter paper. Filter paper possesses all of the qualities required of a bibulous matrix of the present invention, plus the advantages of abundant supply, favorable economics, and a variety of suitable grades. Such paper has been found to be extremely satisfactory for use as a matrix material for suspending and positioning both the separating reagent and the test reagent. Filter paper has been found to have particular utility in retaining the separating reagent, and in this regard the type of filter paper used has been found to influence the effectiveness of the separation process. As known to those skilled in the art of basic chemistry, filter paper can be obtained in a variety of thicknesses and porosities. Since use of the device requires the separating reagent to at least momentarily contact the whole blood and the substantially colorless fluid must be allowed to emerge therefrom, both of the above factors influence the efficiency of the system. When coarse porosity paper is used, the thickness thereof should be sufficient to allow minimal contact time between the whole blood and the separating reagent. The reverse of this is likewise true; and when a fine porosity paper is utilized, a relatively thin sheet may be employed. The proper balance between these factors, as well as a judicious selection of the concentration of separating reagent in the impregnating solution is well within the experimental techniques used by those skilled in the art of preparing test devices such as those described in the present specification.

Table 1 is presented to illustrate the variety of the types of filter paper that are available and the differences in filter paper with respect to paper saturation by blood. The filter papers of Table 1 were tested for speed of blood saturation by placing a drop of whole blood onto the top of a cut paper pad, and recording the time required for the blood to saturate to the bottom side of the paper. A time variance of from 15 sec. to 0.5 sec., for the same thickness paper, indicates that a judicious selection of filter paper will allow one skilled in the art of preparing test devices ready access to the paper of his needs.

TABLE 1

| PENETRATING ABILITY OF BLOOD THROUGH FILTER PAPERS | | | |
|---|---|---|---|
| Paper (Sec) | Uniformity | Thickness (mm) | Time |
| SS3469 | Good | 0.32 | 15 |
| What 3 | Good | 0.38 | 10 |
| SS413 | Good | 0.24 | 10 |
| What 3mm | Good | 0.31 | 8 |
| SS2316 | Fair | 0.34 | 8 |
| SS593A | Fair | 0.33 | 5 |
| What 17 | Good | 0.31 | 3 |
| ED237 | Good | 0.43 | 2 |
| SS591A | Good | 0.18 | 2 |
| SS470 | Fair | 0.88 | 2 |
| SS497 | Good | 0.20 | 2 |
| What31ET | Good | 0.51 | 1.5 |
| SS2668 | Fair | 0.96 | 1.5 |
| ED205 | Fair | 0.69 | 1 |
| SS404 | Fair | 0.20 | 1 |
| Buck245 | Fair | 0.73 | 1 |
| Buck S22 | Fair | 0.59 | 1 |
| SS903 | Good | 0.50 | 1 |
| SS2312 | Fair | 0.30 | 0.5 |
| What90 | Fair | 0.29 | 0.5 |
| Mead624 | Fair-Poor | 0.43 | 0.5 |
| BuckS12 | Good | 0.21 | 0.5 |
| Buck240 | Fair | 0.45 | 0.5 |
| BuckS15 | Fair | 0.38 | 0.5 |
| ED204 | Fair | 0.40 | 0.5 |
| Manufacturers:<br>SS-Schleicher & Schuell, Keene, N.H.<br>What - Whatman Ltd., Maidstone, Kent UK<br>ED - Eaton-Dikeman, Mt. Holy Springs, Mass.<br>Buck - Buckeye, Memphis, Tenn.<br>Mead - Mead, S. Lee, Mass. | | | |

It has been shown that the bibulous matrix of the present invention will not in itself separate the red blood cells from the plasma or serum. If a sample of blood is applied to a nontreated bibulous matrix, no separation is observed as the whole blood permeates through the bibulous matrix. However, if a bibulous matrix is impregnated with a suitable separating reagent, as the blood sample permeates through the bibulous matrix, the red blood cells are removed and fixed in the bibulous matrix as the straw-colored plasma or serum continues to permeate through the bibulous matrix. It has been found that a length of bibulous material at least 2 mm. and preferably at least 3 mm. allows the separating reagent to effectively remove the red blood cells from the serum or plasma.

To achieve the full advantage of the present invention, the bibulous matrix is in the form of a pad, having dimensions of, for example, approximately 0.25 cm by 0.5 cm to 0.5 cm by 1 cm. A pad of these dimensions allows blood to be applied at one end of the pad and have sufficient length for effective red blood cell removal within a reasonably short time. A relatively large sample of blood can be used to increase the speed of serum transfer. However, care should be exercised to avoid the possibility of blood spillover onto the assay area of the device. Increasing the size of the bibulous matrix substantially increases the time of separation, and also requires a much larger blood sample.

The bibulous matrix must include a separating reagent to effect removal of the red blood cells from the plasma or serum. However, the prior art separating reagents, such as the inorganic salts and amino acids disclosed by Fetter in U.S. Patent No. 3,552,925 and 3,552,928 have proven deficient in that they can introduce contaminating ions or molecules to the serum or plasma and they can also separate assayable plasma or serum constituents.

Therefore, in accordance with an important feature of the present invention, an agglutinizing agent, a coagulating agent or a mixture thereof is applied to the bibulous matrix such that the red blood cells of the whole blood sample agglutinate and/or clot as the blood chromatographs through the bibulous matrix. The agglutinated and/or clotted cells become fixed, and are collected within the bibulous matrix, as the plasma or serum continues permeating through the bibulous matrix to the assay area of the device.

As will become more apparent from the following detailed description of the invention, various lectins or thrombin, used individually or in combination, serve to agglutinate and/or coagulate the red blood cells of whole blood, to fix the cells within the bibulous matrix, and to allow the serum or plasma to move relatively unchanged to the assay area of the apparatus. In addition, the lectins and thrombin do not promote excessive hemolysis, such that the red blood cells do not rupture and have their highly colored components mask and interfere with the chromogenic assays.

The lectins are proteins or glycoproteins that are known to agglutinate, or clump, cells and/or precipitate complex carbohydrates. Lectins are isolated from a wide variety of natural sources, including seeds, plant roots, bark, fungi, bacteria, seaweed, sponges, fish eggs, invertebrate and lower vertebrate body fluids, and mammalian cell membranes. The lectins are often blood group specific, and have been used in blood grouping, polyagglutination studies, and various histochemical studies of normal and pathological conditions. The lectins used in the present invention should not be blood group specific, or the general utility of the process and device of the present invention will be limited. Therefore, among the lectins showing no specificity of blood grouping and that are suitable for use in the present invention are lectins from Abrus precatorius (abrin, Jequirty bean), Bauhinia purpurea (camels foot tree), Caragana arborescens (Siberian pea tree), Codium fragile (Green marine algae), Canavalia ensiformis (Con A, Concanavalin A, Jack bean), Glycine max (Soybean), Lathyrus odoratus (Sweet Pea), Lens culinaris (Lentil), Limulus polyphemus (Horseshoe crab, Limulin), Lycopersicon esculentum (Tomato), Maclura pomifera (Osage orange), Mycoplasma gallisepticum, Perseau americana (Avocado), Phaseolus coccineus (Scarlet runner bean), Phaseolus vulgaris (Red Kidney bean), Phytolacca americana (Pokeweed), Pisum sativum (garden pea), Psophocarpus tetragonolobus (winged bean), Ricinus communis (Castor bean), Solanum tuberosum (Potato), Triticum vulgaris (Wheat germ), Vicia faba (fava bean, broad bean), Vigna radiata (Mung bean), Viscum album (European mistletoe), Wisteria floribunda (Japanese wisteria), and other like, nonblood specific lectins.

As will become more apparent hereinafter, the preferred lectins for use in the present invention are the lectin concanavalin A (jack bean), the lectin from solanum tuberosum (potato), the lectin from Triticum vulgaris (wheat germ), the lectin from Bauhinia purpurea (camels foot tree) and the lectin from Phytolacca americana (pokeweed). To achieve the full advantage of the present invention, the bibulous matrix is impregnated with the lectin from potato or the lectin from pokeweed.

In addition to, or in place of, the above-described lectins, a coagulating agent also can be used to effect separation of the serum from the cellular components of whole blood. Specifically, the enzyme thrombin, from bovine plasma, has been used with the bibulous matrix of the present invention to successfully separate serum from whole blood samples. The bovine thrombin effectively promotes blood clotting such that the red blood cells are removed from the whole blood as the blood permeates through the impregnated bibulous matrix. Other thrombins that can be utilized according to the method of the present invention include human thrombin, goat thrombin, pig thrombin and sheep thrombin. It has also been found that it is unnecessary to immobilize the thrombin, or the lectins, onto the bibulous matrix and that effective separation can be effected by using the lectins, the thrombin, or a combination thereof.

As previously described, the device of the present invention includes of one or more bibulous matrices, treated and arranged to separate soluble serum or plasma constituents from undiluted whole blood. The whole blood is deposited on a portion of a bibulous matrix that has been impregnated with a blood separating reagent, such as a nonblood specific lectin and/or a thrombin, whereby the blood cells are separated from the serum or plasma. In accordance with one important embodiment of the present invention, the serum or plasma then proceeds through the bibulous matrix to an assay region that has been previously treated with a suitable testing reagent for a particular assay.

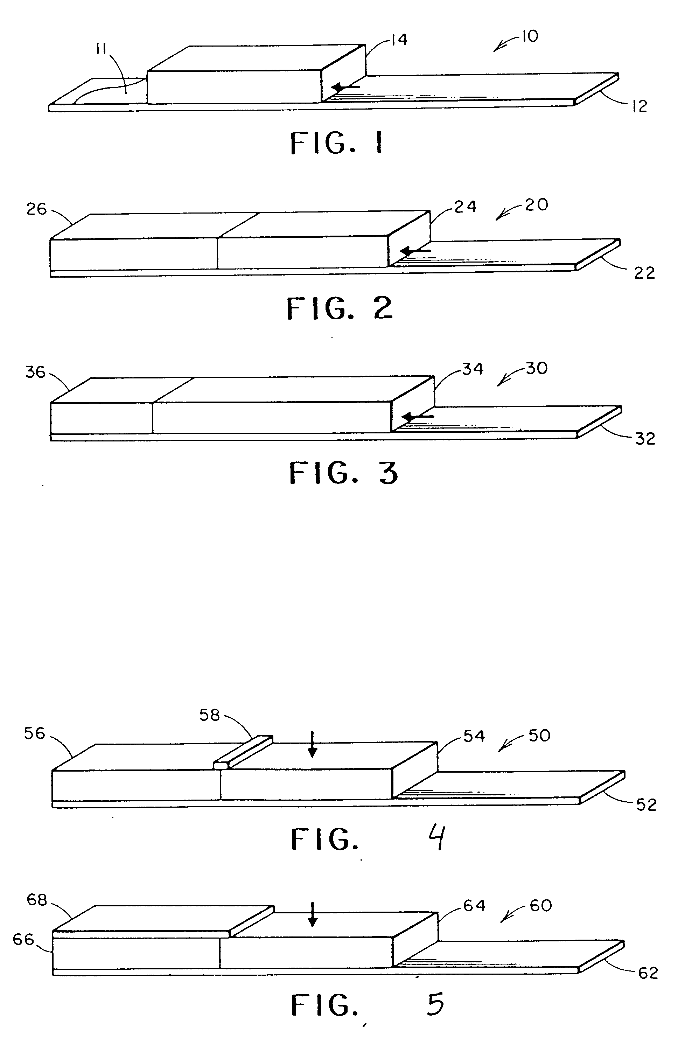

Specifically, the positioning of the bibulous matrices and the separating- and testing-reagents may be better understood by reference to FIGS. 1 through 11. FIG. 1 shows a perspective view of a separating device 10 including a bibulous matrix 14 securely adhered to a support strip or handle 12. Sample is introduced in the direction of the arrow at one end of the separating area of the matrix 14 and a portion thereof 11 passes through said matrix area. FIG. 2 shows a perspective view of a testing device 20 including a first bibulous matrix 24 impregnated with the separating reagent and a second bibulous matrix 26 impregnated with a suitable testing reagent, both bibulous matrices securely adhered to a support strip or handle 22. As will become more apparent hereinafter, in order to facilitate the quantitative determination of plasma or serum constituents, it is preferred that the support strip or handle 22 be manufactured from a hydrophobic material. For the arrangement illustrated in FIG. 2, to achieve the full advantage of the present invention, the whole blood sample should be introduced in the area of the arrow. Such a placement allows the blood to permeate through the separating reagent-impregnated bibulous matrix without spillover of the unseparated whole blood onto the assay area of the device. Spillover of whole blood onto the assay area of the device would interfere with the chromogenic test of the assay area.

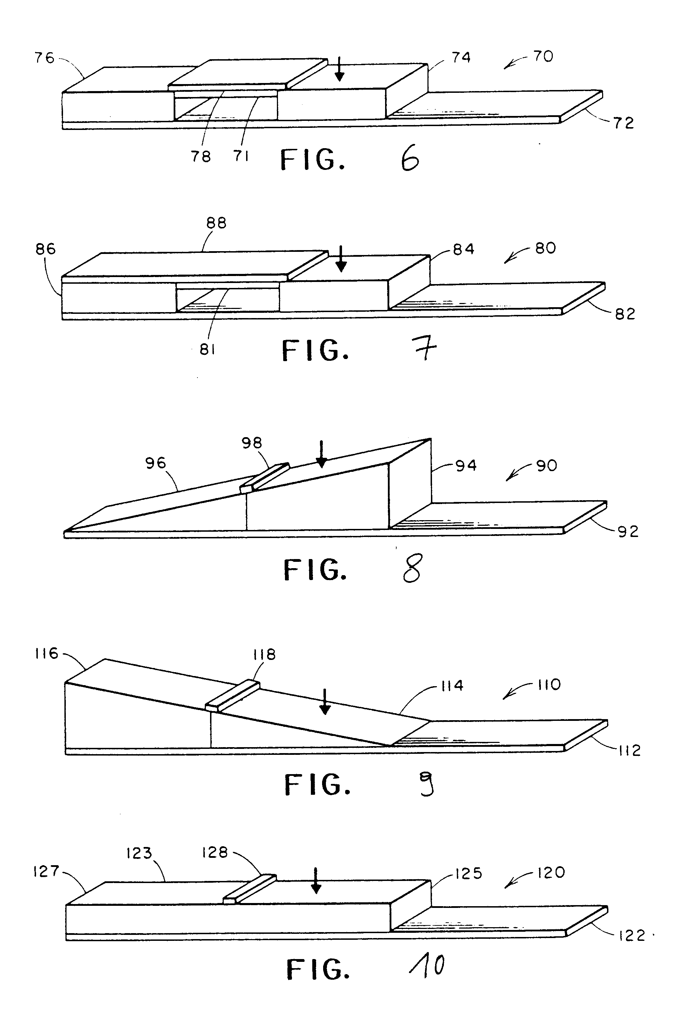

A similar arrangement is seen in FIG. 3, illustrating that the first bibulous matrix 34 and the second bibulous matrix 36 attached to substrate 32 of test device 30. The matrices can vary relatively in size to account for a larger blood sample or for a smaller blood sample. The relative sizes of the bibulous matrices will depend upon the plasma constituent being assayed and how large a blood sample is required to test for that constituent.

FIGS. 4 and 5 are views of devices similar to FIG. 2, with the addition of a hydrophobic barrier 58 (68), made of hydrophobic materials such as plastic or wax disposed between the two bibulous matrices 54 (64) and 56 (66) attached to substrate 52 (62) to prevent whole blood from contacting the assay area of the device 50 (60). The hydrophobic barrier is positioned above the bibulous matrices, near the end of the first bibulous matrix 54 (64) that is in contact with the second bibulous matrix 56 (66). The barrier 58 (68) permits the blood sample to be introduced on top of the device, as indicated by the arrow, without spillover of the sample onto the assay area of the device. If barrier 58 (68) is absent, blood cannot be added to the bibulous matrix 54 (64) too vigorously or blood will run onto bibulous matrix 56 (66) before chromatographing through the separating reagent. Other configurations of the device of the present invention are illustrated in FIGS. 6 and 7, wherein the two bibulous matrices 74 (84) and 76 (86) attached to a substrate 72 (82) are not in intimate contact, but physically separated and connected by a bibulous thin-tissue bridge 71 (81), whereby the plasma or serum can travel from the first bibulous matrix 74 (84) to the second bibulous matrix 76 (86) for assay. A hydrophobic barrier 78 (88) is disposed to cover the thin tissue bridge 71 (81) to avoid contamination of the assay area by the deeply colored whole blood.

FIGS. 8 and 9 are alternate configurations 90 (110) wherein the amount of the undiluted plasma or serum required may be increased or decreased per dose of applied blood by varying the size of the second bibulous matrix 96 (116) relative to the size of the first bibulous matrix 94 (114). Such configurations attached to substrates 92 (112) and containing a barrier 98 (118) allows flexibility in blood assays by making chromogenic reactions more responsive to quantitative determination.

FIG. 10 illustrates a configuration 120 utilizing a single bibulous matrix generally designated 123 attached to a substrate 122. The separating reagent is impregnated in one portion 125 of matrix 123, and the testing reagent is impregnated in another portion 127 of matrix 123. Although a single matrix test device can be constructed as indicated, to achieve the full advantages of the present invention, the device is fabricated such that the separating chemical is impregnated in a first area of the bibulous matrix and the testing reagent is impregnated in a second area of the bibulous matrix. In the configuration there is no possibility of chemical incompatibility or instability between the separating reagent and the testing reagent.

The following Examples and Tables are given for the purpose of illustrating the present invention.

## EXAMPLE 1

## IMPREGNATION OF THE BIBULOUS MATRIX

The agglutinizing agent and/or coagulating agent, such as bovine thrombin, the lectin from solanum tuberosum, the lectin concanavalin A, and/or the lectin from phytolacca americana, is solubilized in normal, or isotonic, saline solution. A bibulous matrix, such as Whatman 31ET or Whatman 3MM filter paper, is then impregnated with the lectin- and/or thrombin-containing saline solution by either immersion or spraying the solution onto sheets or precut strips of the bibulous matrix. The impregnated bibulous matrix is then dried at 50° C. for 20 to 25 minutes, prior to use.

It is not necesary to immobilize the thrombin or lectin when impregnating the bibulous matrix as in Example 1. The simple drying technique is sufficient to maintain the lectin or thrombin in place for separation of the red blood cells. The bibulous matrix, after appropriate sizing, e.g., 0.5 cm x 0.5 cm. is ready to be secured to a transparent or opaque, hydrophobic plastic handle. Immediately adjacent to, and in contact with, the impregnated bibulous matrix, is a second bibulous matrix containing the testing reagents necessary to assay for a particular plasma- or serum-soluble constituent.

Tests to determine the ability of the coagulating agent bovine thrombin and the agglutinizing agents concanavalin A, solanum tuberosum, triticum vulgaris and phytolacca americana to agglutinate or clot blood were run at concentrations ranging from 90 to 900 NIH units/cm$^3$ of thrombin, 140 to 9200 units/cm$^3$ concanavalin A; 4000 to 40000 units/cm$^3$ solanum tuberosum lectin; 115 to 1150 units/cm$^3$ triticum vulgaris lectin; and 57 to 570 units/cm$^3$ phytolacca americana lectin. In each case, a bibulous matrix was impregnated with the separating reagent, and the matrix was securely adhered to a plastic handle or substrate. An untreated pad of filter paper was adhered adjacent to, and in contact with, the impregnated bibulous matrix. For each test the amount of plasma or serum separated from whole blood was determined, and the time of separation noted. The whole blood was deposited on the separating reagent-impregnated bibulous matrix and allowed to move chromatographically through the impregnated bibulous matrix to the untreated substrate. Table II contains the optimum concentration range found for each separating reagent and the percent of separated plasma or serum observed.

TABLE II

| Separating Reagent | Optimum Concentration Range | Percent of Plasma Observed |
|---|---|---|
| Thrombin | 300-450 NIH units/cm$^3$ | 40% |
| Concanavalin A | 600-920 units/cm$^3$ | 40% |
| Solanum tuberosum | 12000-16000 units/cm$^3$ | 100% |
| Phytolacca americana | 570 units/cm$^3$ | 60% |
| Triticum vulgaris | 1150 units/cm$^3$ | 80% |

Identical tests were performed in the presence of calcium chloride or manganese sulfate, since these two salts have been proposed as blood clotting accelerators. However, when used in conjunction with the separating reagents of the present invention, at concentrations of 0.5, 1.0, and 3.0%, no improvements in time of separation or amount of separation was observed. Similarly, to improve the wetting of the bibulous matrix, the anionic surfactant, sodium alpha-olefin sulfonate, was impregnated into the bibulous matrix at 0.1%, in addition to the separating reagent. The wetting of the bibulous matrix was improved, reducing the time for plasma or serum separation, however the amount of separated plasma or serum was not increased. Identical results were observed in utilizing a combination of calcium chloride and sodium alpha-olefin sulfonate with the thrombin or lectin of the present invention.

In accordance with another important feature of the present invention, the separating reagent effectively separated the plasma and serum from the whole blood sample even in the presence of anticoagulants. Specifically, whole blood containing anticoagulants such as heparin or ethylenediaminetetraacetic acid (EDTA) is still amenable to the separation and testing process and device of the present invention. Therefore, fresh blood samples can be treated with anticoagulants and then tested by the process and device of the present invention at a later date. In accordance with another important feature of the present invention, hemolysis is essentially eliminated in separations utilizing lectins and was minimal in separations utilizing thrombin. The degree of lysis in the thrombin examples was such that the coloration from the highly colored red blood cells did not materially interfere in any subsequent assays for plasma-or serum-soluble constituents.

In addition to unexpectedly efficient separation of the red blood cells from the plasma or serum, the process and device of the present invention allow enough plasma or serum to reach the assay areas of the device, and the plasma or serum reaches the assay area of the device with an essentially unaltered composition. Generally, the proper amount of serum or plasma has reached the assay area when the assay area is saturated with plasma or serum. This is accomplished by either using a large enough blood sample to assure plasma or serum saturation of the assay area, or, preferably, adjusting the relative sizes of the separating-reagent impregnated bibulous matrix and the testing reagent-impregnated bibulous matrix such that the assay area will be saturated with plasma or serum - the sizes of bibulous matrices depending upon a predetermined blood sample size and the separating reagent utilized. This process allows for an essentially fixed amount of plasma or serum to reach the assay area, and renders a more accurate soluble constituent determination. The variables of blood sample size, bibulous matrix size, and the amount of testing reagent to impregnate into the assay area bibulous matrix easily can be determined by those skilled in the art after the particular agglutinizer and/or coagulant is chosen as a separating reagent and its separation efficiency determined.

In accordance with another important feature of the present invention, essentially none of the plasma- or serum-soluble constituents of whole blood are separated from the plasma or serum along with the red and white blood cells, nor do intercellular components of the cells contaminate the separated clear liquid. To demonstrate the utility of the present invention, assays were run on the clear liquid that permeated to the assay area after red blood cell separation. Surprisingly, the method and device of the present invention separated the plasma or serum with no increase in potassium ion or loss of cholesterol, essentially no evidence of red blood cells, while precipitating out the white blood cells in addition to the red blood cells, from the whole blood sample. Table III outlines the results of the assays for potassium ion, cholesterol, red blood cells (erythrocytes) and white blood cells (leukocytes) on the serum or plasma obtained after red blood cell separation. Leukocytes and erythrocytes were assayed using test papers sensitive to leukocyte esterase and the peroxidative activity of hemoglobin, whereas cholesterol and potassium test papers were used to assay for those particular constituents. The assays were performed on plasmas or serums obtained by using the separating reagents thrombin, lectins of solanum tuberosum and phytolacca americana, and the agent used in Fetter U.S. Patent No. 3,552,925, namely sodium sulfate, on a fresh whole blood sample.

TABLE III

| PLASMA OR SERUM ASSAYS | | | | |
|---|---|---|---|---|
| Separating Reagent | Leukocytes | Blood | Potassium Ion | Cholesterol |
| Thrombin | Negative | Positive | Positive | Unknown (lysisblocked determination) |
| Solanum tuberosum lectin | Very slight positive | Slight positive | Positive | Positive |
| Phytolacca americana lectin | Slightly positive | Negative | Positive | Positive |
| Sodium sulfate | Inconclusive | Slight positive | Positive | Negative |
| Positive - observable reaction Negative - no observable reaction | | | | |

From the data in Table III, it can be seen that the salts of the Fretter patent precipitate high molecular weight constituents of plasma or serum, such as cholesterol, making them unavailable for testing at the assay area of the device. However, the separating reagents of the present invention do not promote precipitation of high molecular weight constituents. Although thrombin promoted sufficient lysis to block the assay of cholesterol in serum, lysis can be controlled by using normal saline, as opposed to phosphate-buffer saline, when impregnating a bibulous matrix with the thrombin separating reagent solution. Hemolysis inhibiting agents can also be impregnated into the bibulous matrices of the present invention. Therefore, high molecular weight components of plasma or serum can be assayed by using any of the separating reagents of the present invention. Also, in certain instances, hemolysis, or blood staining, may be desirable since assays then can be performed on the constituents present within the red blood cells.

In addition to the fast and efficient separation of the serum or plasma from the cellular constituents of whole blood, and the essentially complete migration of unaltered plasma or serum to the assay area, the method and device of the present invention allow a quantitative assay of a plasma- or serum-soluble constituent without dilution of the whole blood or plasma. Testing the undiluted serum or plasma both omits a manipulative step and, more importantly, eliminates the possibility of technician error. The proper amount of a suitable chromogenic reagent can be impregnated into the assay area for immediate reaction with the freshly separated and undiluted plasma or serum. The extent of the chromogenic reaction, and therefore the quantitative amount of the soluble constituent, can then be determined by the chromogenic detection techniques that are well-known in the art.

For example, the following procedures are indicative of how the quantitative amounts of total bilirubin or creatinine can be determined by the method and device of the present invention.

EXAMPLE 2

DETERMINATION OF TOTAL BILIRUBIN IN PLASMA OR SERUM

An undiluted whole blood sample is applied to a device as illustrated in FIGS. 2 through 9. The sample is deposited in an area of the device containing a thrombin and/or a lectin impregnated on a bibulous matrix (illustrated by an arrow). The blood sample chromatographs through the impregnated bibulous matrix, whereby the red and white blood cells are separated from the plasma or serum. The blood sample is of sufficient size such that after chromatographing through the thrombin-and/or lectin-impregnated bibulous

11

matrix, the amount of serum or plasma is sufficient to completely wet, or saturate, the adjacent assay area. A testing reagent of 0.4% w/w 2,4-dichloroaniline, 1.1% w/w sodium nitrite, 57.2% w/w dyphylline, 35.5% w/w buffer, and 5.8% w/w/nonreactive ingredients, previously impregnated into the assay area of the device reacts with the bilirubin in the serum or plasma to quantitatively determine the total bilirubin in the serum or plasma.

When used with reflectance photometer, the method provides a response to the concentration of total bilirubin in an undiluted sample. The assay method is based on the van den Bergh reaction, modified to use 2,4-dichloroaniline and a diazo coupling accelerator. The final product, azobilirubin, behaves as an indicator, that is red-purple in color under acid conditions. The concentration of total bilirubin in a sample is quantified from a calibration curve.

After an incubation period of 75 seconds, the concentration of total bilirubin in the sample is determined by measuring the change in reflectance at 560 nanometers (nm) with reference to a calibration curve generated using calibrators.

A calibration curve is internally generated. Once the calibration curve has been generated, each test requires approximately 60 μL of whole blood and one device of the present invention. Results are obtained in mg/dL or μmol/l directly from a reflectance photometer. No calculations are required. The test responds from 0.4 to 7.5 mg/dL (7 to 130 μmol/l) serum or plasma total bilirubin. Serum total bilirubin values of 0.1 to 1.2 mg/dL (1.7 to 20.5 μmol/l) have been suggested as the adult normal range.

EXAMPLE 3

DETERMINATION OF CREATININE IN PLASMA OR SERUM

The method described in Example 2 is identically used to quantitatively determine the amount of creatinine in undiluted whole blood samples. However, the testing reagent of this example comprises 43.5% w/w potassium hydroxide, 55.8% w/w 3,5-dinitrobenzoic acid, and 0.6% w/w nonreactive ingredients.

When used with a reflectance photometer, the method provides a direct reading of the concentration of creatinine in the sample. The assay method is based on the Benedict-Behre reaction in which creatinine reacts with 3,5-di-nitrobenzoic acid in an alkaline medium to form a purple-colored complex. The concentration of creatinine in the sample is quantified from a calibration curve generated using calibrators.

During an incubation and test period of 30 seconds, the concentration of creatinine in the sample is determined by measuring the rate of change in reflectance at 560 nm with reference to a calibration curve generated using calibrators. The result is displayed digitally by a reflectance photometer.

A calibration curve is internally generated. Once the calibration curve has been generated, each test requires approximately 60 μL of whole blood sample and one device of the present invention. By using a reflectance photometer, no calculations are required. The test covers a range of 0 to 15 mg/dL (0 to 1326 μmol/l serum or plasma creatinine. Serum creatinine values of 0.6 to 1.2 mg/dL (53 to 106 μmol/l) for males and 0.5 to 1.0 mg/dL (44 to 88 μmol/l) for females have been suggested as the normal range. Nevertheless, the range afforded is large enough to test populations several times higher than the normal suggested range.

With the appropriate chemistry, assays on undiluted plasma or serum, utilizing known chromogenic reactions and a reflectance spectrophotometer, can also be made for uric acid, potassium ions, glucose, galactose, urea, phenylalanine, cholesterol, triglycerides, etc., and various enzymes. See, for example, U.K. Patent 2,014,155; U.S. 4,186,251; U.S. 4,057,394; etc., which references are hereby incorporated by reference.

EXAMPLE 4

Two 0.5 centimeter square pads of filter paper were applied in side-by-side configuration to a polystyrene substrate. The first pad was made from a thick (0.88 mm) and very absorbent paper such as Schleicher and Schuell Number 470 paper (S and S). The other pad was made from Eaton Dikeman Number 204 paper. The S and S paper allows lectin to be absorbed and plasma to escape once agglutination occurs. The paper also permits a larger volume of sample to contact with the lectin. This is advantageous since if too small a sample volume interreacts with lectin, not enough plasma is generated to saturate the reagent area. In addition to the lectin present in the S and S paper, the paper contained 3 percent by weight of PVP K30 polyvinylpyrrolidone (Luviskol). The polyvinylpyrrolidone slows the flow of the blood into the pad and allows sufficient time for lectin agglutination to occur before the blood permeates into the adjoining Eaton Dikeman pad. Moreover, use of PVP in S and S paper eliminates the necessity for

12

employing hydrophobic barrier 98 in FIG. 8.

Using this configuration, the time necessary to saturate the Eaton Dikeman 204 paper was one minute without reagents present in the Eaton Dikeman paper and two minutes for saturation when reagents are present for the determination of potassium.

In general, from 1 to 5 percent by weight, and preferably from 2 to 4 percent by weight of PVP will bring about the desired delay in the rate of the entry of blood and thereby bring about good separation. Other separation promoters which have this unique capability for promoting effective separation by slowing up the rate of the entry of blood include Gantrez AN119, a copolymer of methylvinylether and maleic anhydride; Gelvatol 20/30, polyvinyl alcohol; and Reten 220, a cationic acrylamide copolymer.

## Claims

1. A method of separating plasma or serum from an undiluted whole blood sample comprising contacting the whole blood sample with a bibulous matrix, said bibulous matrix containing a sufficient amount of a blood cell agglutinizing agent, or a blood cell coagulating agent or mixture thereof to agglutinize and/or coagulate cellular components from the whole blood sample in the area of the bibulous matrix, without dilution of the whole blood, while allowing a quantity of the plasma or serum portion of the whole blood sample to flow through the bibulous matrix separate from the cellular components of the whole blood, wherein the agglutinizing agent is a nonblood group specific lectin and the coagulating agent is a thrombin.

2. A method of separating plasma or serum from an undiluted whole blood sample and testing the plasma or serum for a soluble constituent comprising contacting the whole blood with a first bibulous matrix, said first bibulous matrix containing a sufficient amount of an agglutinizing agent, coagulating agent or mixture thereof to agglutinize and/or coagulate the cellular components from the whole blood thereby separating the plasma or serum from the cellular components of the whole blood, allowing the separated plasma or serum to contact a second bibulous matrix which is positioned side-by-side to the first bibulous matrix containing a suitable testing reagent for a soluble constituent of the plasma or serum, said second bibulous matrix being sufficiently close to the first bibulous matrix for contact of said separated serum or plasma with said second bibulous matrix to produce an interaction between a predetermined component of the second bibulous matrix and the testing reagent resulting in a detectable change in the second bibulous matrix, and detecting the detectable change in said second bibulous matrix in an area of the second bibulous matrix spaced from the separated cellular components of the whole blood, wherein the agglutinizing agent is a nonblood group specific lectin selected from the group consisting of Abrus precatorius (abrin, Jequirty bean), Bauhinia purpurea (camels foot tree), Caragana arborescens (Siberian pea tree), Codium fragile (Green marine algae), Canavalia ensiformis (Con A, Concanavalin A, Jack bean), Glycine max (Soybean), Lathyrus odoratus (Sweet Pea), Lens culinaris (Lentil), Limulus polyphemus (Horseshoe crab, Limulin), Lycopersicon esculentum (Tomato), Maclura pomifera (Osage orange), Mycoplasma gallisepticum, Perseau americana (Avacado), Phaseolus coccineus (Scarlet runner bean), Phaseolus vulgaris (Red Kidney bean), Phytolacca americana (Pokeweed), Pisum sativum (garden pea), Psophocarpus tetragonolobus (winged bean), Ricinus communis (Castor bean), Solanum tuberosum (Potato), Triticum vulgaris (Wheat germ), Vicia faba (fava bean, broad bean), Vigna radiata (Mung bean), Viscum album (European mistletoe), Wisteria floribunda (Japanese wisteria), and mixtures thereof, and the coagulating agent is a thrombin.

3. The method of claim 2 wherein the first bibulous matrix and the second bibulous matrix are the same or different hydrophilic materials selected from the group consisting of hydrophilic inorganic powders, sponge materials, argillaceous materials, cloth, hydrophilic naturally-occurring polymers, hydrophilic naturally-occurring modified polymers, hydrophilic synthetic polymers, diatomaceous earth, cellulosic materials, and mixtures thereof.

4. The method of claim 2 in which a separation promoter is added to the first bibulous matrix.

5. The method of claim 4 in which the separation promoter is polyvinyl pyrrolidone.

6. A device for separating plasma or serum from whole blood and for detecting a soluble constituent of plasma or serum comprising a first bibulous matrix, containing
   a sufficient amount of an agglutinizing agent, or a coagulating agent or mixture thereof, to remove the cellular components of whole blood upon contact therewith, and a second bibulous matrix containing a suitable testing reagent in a sufficient quantity for interaction with a soluble constituent of the plasma or serum to produce a detectable change in the second bibulous matrix upon contact with the serum or plasma, said second bibulous matrix being positioned side-by side to the first bibulous matrix and so positioned and sized that the interaction between the soluble constituent of the plasma or serum and the testing reagent is detected without substantial interferences from the cellular material separated by the first bibulous matrix, wherein the agglutinizing agent is a nonblood group specific lectin selected from Abrus precatorius (abrin, Jequirty bean), Bauhinia purpurea (camels foot tree), Caragana arborescens (Siberian pea tree), Codium fragile (Green marine algae), Canavalia ensiformis (Con A, Concanavalin A, Jack bean), Glycine max (Soybean), Lathyrus odoratus (Sweet Pea), Lens culinaris (Lentil), Limulus polyphemus (Horseshoe crab, Limulin), Lycopersicon esculentum (Tomato), Maclura pomifera (Osage orange), Mycoplasma gallisepticum, Perseau americana (Avacado), Phaseolus coccineus (Scarlet runner bean), Phaseolus vulgaris (Red Kidney bean), Phytolacca americana (Pokeweed), Pisum sativum (garden pea), Psophocarpus tetragonolobus (winged bean), Ricinus communis (Castor bean), Solanum tuberosum (Potato), Triticum vulgaris (Wheat germ), Vicia faba (fava bean, broad bean), Vigna radiata (Mung bean), Viscum album (European mistletoe), Wisteria floribunda (Japanese wisteria), and mixtures thereof, and the coagulating agent is a thrombin.

**Revendications**

1. Procédé pour séparer du plasma ou du sérum d'un échantillon de sang entier non dilué, consistant à mettre en contact l'échantillon de sang entier avec une matrice absorbante, ladite matrice absorbante contenant une quantité suffisante d'un agent d'agglutination des globules sanguins ou d'un agent de coagulation des globules sanguins, ou bien d'un de leurs mélanges, pour provoquer l'agglutination et/ou la coagulation des constituants cellulaires de l'échantillon de sang entier dans la zone de la matrice absorbante, sans dilution du sang entier, tout en laissant une quantité de la portion de plasma ou de sérum de l'échantillon de sang entier s'écouler à travers la matrice absorbante, séparément des constituants cellulaires du sang entier, dans lequel l'agent d'agglutination est une lectine non spécifique d'un groupe sanguin et l'agent de coagulation est une thrombine.

2. Procédé pour séparer du plasma ou du sérum d'un échantillon de sang entier non dilué et pour tester le plasma ou le sérum pour la détermination d'un constituant soluble, consistant à mettre en contact le sang entier avec une première matrice absorbante, ladite première matrice absorbante contenant une quantité suffisante d'un agent d'agglutination ou d'un agent de coagulation, ou bien d'un de leurs mélanges, pour provoquer l'agglutination et/ou la coagulation des constituants cellulaires du sang entier, ce qui permet de séparer le plasma ou le sérum des constituants cellulaires du sang entier, à laisser le plasma ou sérum séparé entrer en contact avec une seconde matrice absorbante qui est située côte à côte avec la première matrice absorbante contenant un réactif analytique convenable pour la détermination d'un constituant soluble du plasma ou du sérum, ladite seconde matrice absorbante étant suffisamment proche de la première matrice absorbante pour la mise en contact dudit sérum ou plasma séparé avec ladite seconde matrice absorbante pour provoquer une interaction entre un constituant prédéterminé de la seconde matrice absorbante et le réactif analytique avec pour résultat une variation détectable dans la seconde matrice absorbante, et à détecter la variation détectable dans ladite seconde matrice absorbante dans une zone de la seconde matrice absorbante distante des constituants cellulaires séparés du sang entier, dans lequel l'agent d'agglutination est une lectine non spécifique d'un groupe sanguin choisie entre les lectines de Abrus precatorius (abrine, pois d'Amérique), Bauhinia purpurea (arbre "patte de chameau"), Caragana arborescens (arbre aux pois), Codium fragile (algue marine verte), Canavalia ensiformis (Con A, Concanavaline A, haricot sabre), Glycine max (soja), Lathyrus odoratus (gesse odorante), Lens culinaris (Lentille), Limulus polyphemus (crabe des Moluques, limule), Lycopersicon esculentum (tomate), Maclura pomifera (oranger des Osages), Mycoplasma gallisepticum, Persea americana (avocatier), Phaseolus coccineus (haricot d'Espagne), Phaseolus vulgaris (haricot à rames), Phytolacca americana (raisin d'Amérique), Pisum sativum (pois potager), Psophocarpus tetragonolobus (pois carré), Ricinus communis (ricin), Solanum tuberosum (pomme de terre), Triticum vulgaris (blé, germes), Vicia faba (fève commune, grosse fève), Vigna radiata (ambérique), Viscum album (gui d'Europe), Wisteria floribunda (Wisteria japonaise), et leurs mélanges, et l'agent de coagulation est une thrombine.

3. Procédé suivant la revendication 2, dans lequel la première matrice absorbante et la seconde matrice

14

absorbante sont constituées de matières hydrophiles identiques ou différentes choisies dans le groupe comprenant des poudes inorganiques hydrophiles, des matières du type éponge, des matières argileuses, une étoffe, des polymères hydrophiles naturels, des polymères hydrophiles naturels modifiés, des polymères hydrophiles synthétiques, la terre de diatomées, des matières cellulosiques et leurs mélanges.

4. Procédé suivant la revendication 2, dans lequel un activateur de séparation est ajouté à la première matrice absorbante.

5. Procédé suivant la revendication 4, dans lequel l'activateur de séparation est la polyvinylpyrrolidone.

6. Dispositif pour séparer le plasma ou le sérum du sang entier et pour détecter un constituant soluble du plasma ou du sérum, comprenant une première matrice absorbante, contenant
une quantité suffisante d'un agent d'agglutination ou d'un agent de coagulation, ou bien d'un de leurs mélanges, pour l'élimination des constituants cellulaires du sang entier par contact avec cet agent, et une seconde matrice absorbante contenant un réactif analytique convenable en une quantité suffisante pour l'interaction avec un constituant soluble du plasma ou du sérum pour la production d'une variation détectable dans la seconde matrice absorbante lors du contact avec le sérum ou le plasma, ladite seconde matrice absorbante étant placée côte à côte avec la première matrice absorbante et présentant une position et des dimensions telles que l'interaction entre le constituant soluble du plasma ou du sérum et le réactif analytique soit détectée sans interférences notables provoquées par la matière cellulaire séparée par la première matrice absorbante, dans lequel l'agent d'agglutination est une lectine non spécifique d'un groupe sanguin, choisie entre les lectines de Abrus precatorius (abrine, pois d'Amérique), Bauhinia purpurea (arbre "patte de chameau"), Caragana arborescens (arbre aux pois), Codium fragile (algue marine verte), Canavalia ensiformis (Con A, Concanavaline A, haricot sabre), Glycine max (soja), Lathyrus odoratus (gesse odorante), Lens culinaris (lentille), Limulus polyphemus (crabe des Moluques, limule), Lycopersicon esculentum (tomate), Maclura pomifera (oranger des Osages), Mycoplasma gallisepticum, Persea americana (avocatier), Phaseolus coccineus (haricot d'Espagne), Phaseolus vulgaris (haricot à rames), Phytolacca americana (raisin d'Amérique), Pisum sativum (pois potager), Psophocarpus tetragonolobus (pois carré), Ricinus communis (ricin), Solanum tuberosum (pomme de terre), Triticum vulgaris (blé, germes), Vicia faba (fève commune, grosse fève), Vigna radiata (ambérique), Viscum album (gui d'Europe), Wisteria floribunda (Wisteria japonaise), et leurs mélanges, et l'agent de coagulation est une thrombine.

## Patentansprüche

1. Verfahren zum Trennen von Plasma oder Serum aus einer unverdünnten Vollblutprobe, umfassend das Inkontaktbringen der Vollblutprobe mit einer saugfähigen Matrix, wobei die saugfähige Matrix eine ausreichende Menge eines Blutzellen agglutinisierenden Mittels oder eines Blutzellen koagulierenden Mittels oder eine Mischung davon enthält, um die zellulären Komponenten aus der Vollblutprobe im Bereich der saugfähigen Matrix zu agglutinisieren und/oder zu koagulieren, ohne das Vollblut zu verdünnen, während man eine Menge des Plasma- oder Serumanteils der Vollblutprobe durch die saugfähige Matrix zur Trennung von den zellulären Bestandteilen des Vollblutes fliessen lässt, worin das agglutinisierende Mittel ein nicht blutgruppenspezifisches Lectin und das Koagulationsmittel Thrombin ist.

2. Verfahren zum Trennen von Plasma oder Serum aus einer unverdünnten Vollblutprobe und Testen des Plasmas oder Serums auf einen löslichen Bestandteil, umfassend das Inkontaktbringen des Vollblutes mit einer ersten saugfähigen Matrix, wobei die erste saugfähige Matrix eine ausreichende Menge eines agglutinisierenden Mittels, eines koagulierenden Mittels oder eine Mischung davon zur Agglutinisierung und/oder Koagulierung der zellulären Bestandteile aus dem Vollblut enthält, wodurch das Plasma oder Serum von den zellulären Betandteilen des Vollblutes abgetrennt wird, und man das abgetrennte Plasma oder Serum in Kontakt mit einer zweiten saugfähigen Matrix kommen lässt, die Seite an Seite der ersten saugfähigen Matrix angeordnet ist, und ein geeignetes Testreagens für einen löslichen Bestandteil des Plasmas oder Serums enthält, wobei die zweite saugfähige Matrix ausreichend eng an die erste saugfähige Matrix für einen Kontakt des abgetrennten Serums oder Plasmas mit der zweiten saugfähigen Matrix angeordnet ist, um eine Wechselwirkung zwischen einem bestimmten Bestandteil der zweiten saugfähigen Matrix und dem Testagens herzustellen, was eine nachweisbare Veränderung in der zweiten saugfähigen Matrix bewirkt, und Bestimmen der nachweisbaren Veränderung in der zweiten saugfähigen Matrix in einem Bereich der zweiten saugfähigen Matrix, welcher getrennt ist von dem abgetrennten zellulären Bestandteil des Vollblutes, wobei das agglutinisierende Mittel ein nicht blutgruppenspezifisches Lectin, ausgewählt aus der Gruppe, bestehend aus Abrus precatorius (Abrin, Paternostererbse), Bauhinia purpurea (Kamelfussbaum), Caragana arborescens (sibirischer Erbsenstrauch), Codium fragile (Grüne Meeralge), Canavalia ensiformis (Con A, Concanacalin A, Jackbohne), Glycine max (Sojabohne), Lathyrus odoratus (Gartenwicke), Lens culinaris (Linse), Limulus polyphemus (Molukkenkrebs, Limulin), Lycopersicon esculentum (Tomate), Maclura pomifera (Osage-Orange), Mycoplasma gallisepticum, Perseau americana (Avacado), Phaseolus coccineus (Scharlachfeuerbohne), Phaseolus vulgaris (Feuerbohne), Phytolacca americana (Kermesbeere), Pisum sativum (Gartenerbse), Psophocarpus tetragonolobus (Flügelbohne), Ricinus communis (Castornuss), Solanum tuberosum (Kartoffel), Triticum vulgaris (Weizenkeimling), Vicia faba (Saubohne), Vigna radiata (Mungbohne), Viscum album (europäische Mistel), Wisteria floribunda (japanische Wisterie) und Mischungen davon, und das koagulierende Mittel Thrombin ist.

3. Verfahren nach Anspruch 2, worin die erste saugfähjige Matrix und die zweite saugfähige Matrix gleiche oder verschiedene hydrophile Materialien sind, ausgewählt aus der Gruppe, bestehend aus hydrophilen anorganischen Pulvern, Schwammaterialien, tonhaltigen Materialien, Gewebe, hydrophilen natürlich vorkommenden Polymeren, hydrophilen natürlich vorkommenden, modifizierten Polymeren, hydrophilen synthetischen Polymeren, Diatomeenerde, Zellulosematerialien und Mischungen davon.

4. Verfahren nach Anspruch 2, wobei ein Trennungsbeschleuniger der ersten saugfähigen Matrix zugegeben wird.

5. Verfahren nach Anspruch 4, wobei der Trennungsbeschleuniger Polyvinylpyrrolidon ist.

6. Vorrichtung zur Trennung von Plasma oder Serum aus Vollblut und Nachweis eines löslichen Bestandteils des Plasmas oder Serums, umfassend eine erste saugfähige Matrix, enthaltend eine ausreichende Menge eines agglutinisierenden Mittels oder koagulierenden Mittels oder Mischungen davon, um die zellulären Bestandteile des Vollblutes nach Inkontaktbringen damit zu entfernen, und eine zweite saugfähige Matrix, enthaltend ein geeignetes Nachweisreagens in einer ausreichenden Menge zur Wechselwirkung mit einem löslichen Bestandteil des Plasmas oder Serums, um eine nachweisbare Veränderung in der zweiten saugfähigen Matrix nach Kontakt mit dem Serum oder Plasma hervorzurufen, wobei die zweite saugfähige Matrix Seite an Seite der ersten saugfähigen Matrix angeordnet ist, und so angeordnet und in der Grösse ausgewählt ist, dass die Wechselwirkung zwischen dem löslichen Bestandteil des Plasmas oder Serums und dem Testmittel ohne wesentliche Beeinträchtigung durch das von der ersten saugfähigen Matrix abgetrennte zelluläre Material nachgewiesen wird, worin das agglutinisierende Mittel ein nicht blutgruppenspezifisches Lectin, ausgewählt aus der Gruppe, bestehend aus Abrus precatorius (Abrin, Paternostererbse), Bauhinia purpurea (Kamelfussbaum), Caragana arborescens (sibirischer Erbsenstrauch), Codium fragile (Grüne Meeralge), Canavalia ensiformis (Con A, Concanacalin A, Jackbohne), Glycine max (Sojabohne), Lathyrus odoratus (Gartenwicke), Lens culinaris (Linse), Limulus polyphemus (Molukkenkrebs, Limulin), Lycopersicon esculentum (Tomate), Maclura pomifera (Osage-Orange), Mycoplasma gallisepticum, Perseau americana (Avacado), Phaseolus coccineus (Scharlachfeuerbohne), Phaseolus vulgaris (Feuerbohne), Phytolacca americana (Kermesbeere), Pisum sativum (Gartenerbse), Psophocarpus tetragonolobus (Flügelbohne), Ricinus communis (Castornuss), Solanum tuberosum (Kartoffel), Triticum vulgaris (Weizenkeimling), Vicia faba (Saubohne), Vigna radiata (Mungbohne), Viscum album (europäische Mistel), Wisteria floribunda (japanische Wisterie) und Mischungen davon, und das koagulierende Mittel Thrombin ist.

FIG. 1

FIG. 2

FIG. 3

FIG. 4

FIG. 5

FIG. 6

FIG. 7

FIG. 8

FIG. 9

FIG. 10